# EUROPÄISCHE PATENTANMELDUNG

(11) **EP 0 879 588 A2**
(43) Veröffentlichungstag der Anmeldung: **25.11.1998**
(21) Anmeldenummer: 98107395.0
(22) Anmeldetag: 23.04.1998
(51) Int. Cl.: A61G 9/02

(54) **Spül- und Desinfektionsvorrichtung für Pflegegeschirr, wie z.B. Bettpfannen, Urinflaschen oder dergleichen**

(30) Priorität: 21.05.1997 DE 29708942 U
(71) Anmelder: Discher Sanitätstechnik GmbH, 42781 Haan (DE)
(72) Erfinder: Discher, Josef, 42781 Haan (DE)
(74) Vertreter: Grundmann, Dirk, Dr.

(57) **Zusammenfassung**

Die Erfindung betrifft eine Spül- und Desinfektionsvorrichtung für Pflegegeschirr, wie z.B. Bettpfannen, Urinflaschen oder dergleichen, mit einer das Pflegegeschirr aufnehmenden Kammer (1), in welche Spülwasser und Dampf zugeführt werden, wobei die Wasserzufuhr magnetventilgesteuert zu- und abschaltbar ist und der Dampf von einem Dampferzeuger (D) erzeugt wird, und schlägt zur Erzielung eines einfacheren und kostensparenderen Aufbaus vor, daß eine kombinierte Spülwasser- und Dampfzufuhrleitung (25) eine unmittelbar oberhalb des Dampferzeuger (D) angeordnete Verzweigung (21) zum Dampferzeuger (D) besitzt.

## Beschreibung

Die Erfindung betrifft eine Spül- und Desinfektionsvorrichtung für Pflegegeschirr, wie z.B. Bettpfannen, Urinflaschen oder dergleichen, mit einer das Pflegegeschirr aufnehmenden Kammer, in welche Spülwasser und Dampf zugeführt werden, wobei die Wasserzufuhr magnetventilgesteuert zu- und abschaltbar ist und der Dampf von einem Dampferzeuger erzeugt wird.

Bei bekannten Spül- und Desinfektionsvorrichtungen der vorgenaunten Art haben sowohl das Spülwasser als auch der Dampf je ein eigenes Leitungssystem mit den zugehörigen zu steuernden Magnetventilen.

Dem Gegenstand der Erfindung liegt die Aufgabe zugrunde, eine Spül- und Desinfektionsvorrichtung der in Rede stehenden Art in ihrem Aufbau einfacher und kostensparender auszugestalten.

Diese Aufgabe ist zunächst und im wesentlichen bei einer Spül- und Desinfektionsvorrichtung mit den Merkmalen des Anspruchs 1 gelöst, wobei darauf abgestellt ist, daß eine kombinierte Spülwasser- und Dampfzufuhrleitung eine unmittelbar oberhalb des Dampferzeugers angeordnete Verzweigung zum Dampferzeuger besitzt.

Die Unteransprüche betreffen vorteilhafte Weiterbildungen der erfindungsgemäßen Lösung.

Zufolge derartiger Ausgestaltung ist eine gattungsgemäße Spül- und Desinfektionsvorrichtung von einfachem, kostensparendem Aufbau angegeben, und zwar ohne Beeinträchtigung der Reinigungsfunktion. Das Spülwasser und der Dampf gelangen nun durch eine kombinierte Spülwasser- und Dampfzufuhrleitung zur das Pflegegeschirr aufnehmenden Kammer, so daß neben der Einsparung einer gesonderten Versorgungsleitung auch ein zusätzliches Magnetventil entfallen kann. Die Reinigung des Pflegegeschirrs erfolgt in der Weise, daß zunächst der Spülvorgang vorgenommen wird. Auschließend wird dann nach Abschalten der Spülwasserzufuhr Dampf aus dem Dampferzeuger über die Spülwasser- und Dampfzuführleitung in die Kammer geleitet einhergehend mit einer thermischen Desinfektion des Pflegegeschirrs. In der Praxis sieht dies so aus, daß erst nach Befüllen des Dampferzeugers Spülwasser über die Verzweigung über die Spülwasser- und Dampfzuführleitung das Spülwasser in die Kammer geleitet wird. Nach Abschalten der Spülwasserzufuhr und Einschalten des Dampferzeugers gelangt dann über ein und dieselbe Verzweigung aus dem Dampferzeuger der Dampf über die Spülwasser- und Dampfzuführleitung in die Kammer. Um eine größere Anzahl von Pflegegeschirren in der Kammer reinigen zu können, sind zwei getrennt voneinander zu- und abschaltbare Wasserzufuhrleitungen vorgesehen, von denen die eine die Verzweigung zum Dampferzeuger besitzt und die andere ummittelbar in ein oberhalb der Kammer angeordnetes Spülaggregat mündet. Hierdurch ist es möglich, ausschließlich durch die zum Spülaggregat führende Wasserzufuhrleitung Spülwasser zuzuführen, um bspw. mit dem Spülaggregat Bettpfannen zu reinigen. Die andere Wasserzufuhrleitung bleibt dann gesperrt. Alternativ können auch beide Wasserzufuhrleitungen geöffnet sein. Dies wird vorgenommen zum gleichzeichtigen Spülen von Bettpfannen und Urinflaschen. Weiterhin ist es möglich, die zum Spülaggregat führende Wasserzufuhrleitung abzuschalten und lediglich die andere Wasserzufuhrleitung zu öffnen, welche über die Verzweigung zum Dampferzeuger mündet. Dieser Zustand wird vornehmlich gewählt, wenn nur Urinflaschen gespült werden sollen. Werden beide Wasserzufuhrleitungen geschlossen durch entsprechendes Betätigen der zugehörigen Ventile, kann über den Dampferzeuger die Desinfektion des Pflegegeschirrs vorgenommen werden. Die eine Wasserzufuhrleitung mündet dabei in Spritzdüsen zur Urinflaschen-Reinigung. Auch ist diese eine Wasserzufuhrleitung strömungsverbunden mit vorzugsweise im oberen Bereich der Kammer befindlichen Dampfdüsen, so daß sämtliches in der Kammer befindliches Reinigungsgeschirr der thermischen Desinfektion ausgesetzt ist. Um den Spül- und Desinfektionsvorgang in der richtigen Reihenfolge vornehmen zu können, werden die Magnetventile von einer elektronischen Zeitsteuereinrichtung betätigt. Weiterhin ist hervorzuheben, daß das Wasser zum Spülen und zur Dampferzeugung einem Wasservorratsbehälter entnommen wird. Ein direkter Leitungsanschluß an das öffentliche Versorgungsnetz entfällt demzufolge, so daß mit Sicherheit verhindert ist, daß eventuell Krankheitskeime in das Versorgungsnetz gelangen. Schließlich ist noch hervorzuheben, daß die Verzweigung ein U-förmiges Durchflußstück ausbildet, wobei der abflußseitge U-Schenkel U-stegseitig einen in den Dampferzeuger mündenden Stutzen ausbildet.

Nachstehend wird ein Ausführungsbeispiel der Erfindung anhand der Zeichnungen erläutert. Es zeigt:
- Fig. 1: eine schematische Darstellung der Spül- und Desinfektionsvorrichtung mit zugehörigem Versorgungssystem, und zwar bei geöffneten Magnetventilen zweier Wasserzufuhrleitungen zwecks eines gleichzeitigen Spülens einer Bettpfanne sowie dreier Urinflaschen,

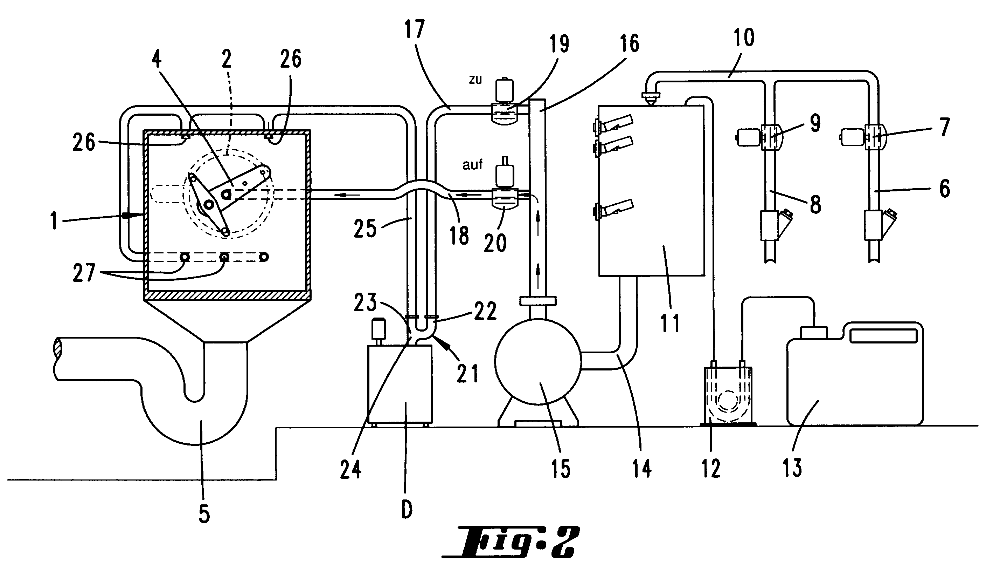

- Fig. 2: eine der Fig. 1 entsprechende Darstellung, wobei nur das Magnetventil der Wasserzufuhrleitung geöffnet ist, welche unmittelbar in ein innerhalb der Kammer angeordnetes Spülaggregat mündet zur Reinigung ausschließlich einer Bettpfanne,
- Fig. 3: ebenfalls eine vergleichbare Darstellung, und zwar bei geöffnetem Magnetventil der Wasserzufuhrleitung, welche über die Verzweigung sowohl zum Dampferzeuger als auch zur kombinierten Spülwasser- und Dampfzufuhrleitung führt zwecks Spülung von Urinflaschen und

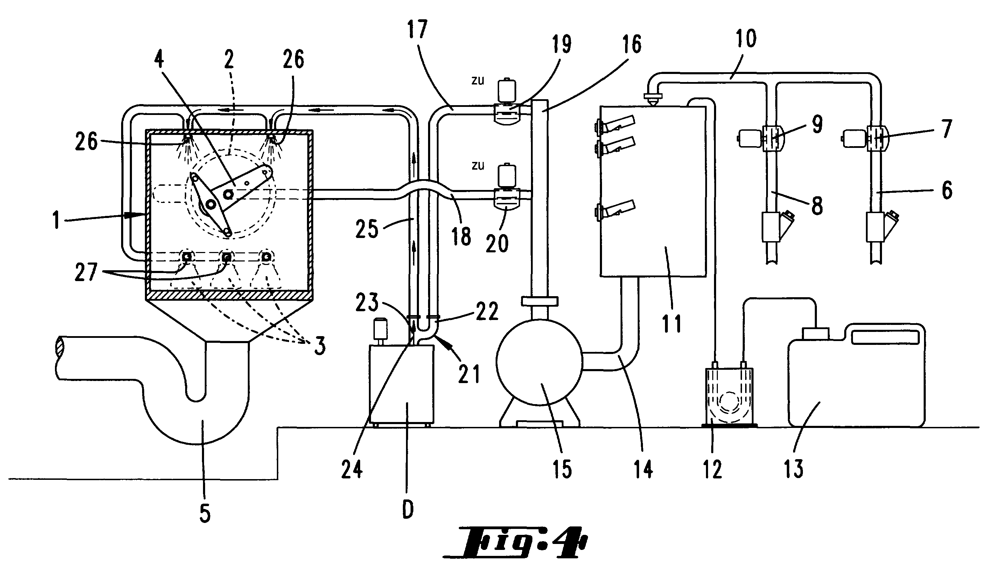

- Fig. 4: die Spül- und Desinfektionsvorrichtung in der Position, in welcher die Magnetventile der Wasserzufuhrleitungen geschlossen sind, während über die Spülwasser- und Dampfzufuhrleitung aus dem Dampferzeuger Dampf in die Kammer geleitet wird zwecks Desinfektion einer Bettpfanne sowie dreier Urinflaschen.

Die dargestellte Spül- und Desinfektionsvorrichtung besitzt eine türverschließbare Kammer 1 zur Aufnahme von Pflegegeschirr, wie z.B. eine strichpunktiert veranschaulichte Bettpfanne 2 sowie beim Ausführungsbeispiel dreier Urinflaschen 3. Die Bettpfanne 2 wird vor einem innerhalb der Kammer 1 rotierbaren Spülaggregat 4 eingehängt, welches mit nicht näher bezeichneten Sprühdüsen versehen ist, um den ganzen Bettpfannenbereich zu erfassen. Unterhalb des Spülaggregates sind nicht näher bezeichnete Aufnahmen für die Urinflaschen 3 vorhanden. Das anfallende Abwasser wird über einen unteren Abfluß 5 der Kammer 1 entsorgt.

Der Spül- und Desinfektionsvorrichtung ist ein Versorgungssystem vorgeordnet. Dieses besitzt eine Kaltwasser-Versorgungsleitung 6 mit integriertem Magnetventil 7 sowie eine Warmwasser-Versorgungsleitung 8 mit zugehörigem Magnetventil 9. Beide Versorgungsleitungen 6, 8 vereinigen sich in einem Mischrohr 10, von welchem das Spülwasser in einen Wasservorratsbehälter 11 einfließt. Zwecks Durchführung einer chemischen Desinfektion steht der Wasservorratsbehälter 11 über eine Dosierpumpe 12 mit einem Kanister 13 in Leitungsverbindung, welcher Kanister 13 ein chemisches Desinfektionsmittel enthält.

Eine vom Behälterboden ausgehende Abflußleitung 14 führt zu einer Druckerhöhungspumpe 15, von welcher das Spülwasser mit Druck in ein Zentralrohr 16 geleitet wird. Von diesem sind zwei Wasserzufuhrleitungen 17, 18 abgezweigt. Jede dieser Wasserzufuhrleitungen 17, 18 ist getrennt voneinander durch ein Magnetventil 19, 20 zu- und abschaltbar. Die eine Wasserzufuhrleitung 17 führt zu einer unmittelbar oberhalb eines Dampferzeugers D angeordneten Verzweigung 21. Diese ist als U-formiges Durchflußstück ausgestaltet derart, daß der zuflußseitige U-Schenkel 22 in die Wasserzufuhrleitung 17 übergeht, während der abflußseitige U-Schenkel 23 U-stegseitig einen in den Dampferzeuger D mündenden Stutzen 24 ausbildet. In Gegenüberlage zum Stutzen 24 setzt sich der abflußseitge U-Schenkel 23 in eine kombinierte Spülwasser- und Dampfzufuhrleitung 25 fort, welche strömungsverbunden mit im oberen Bereich der Kammer 1 befindlichen Dampfdüsen 26 ist. Diese sind so beschaffen, daß durch sie einerseits Dampf und andererseits auch Spülwasser hindurchtreten kann. Ferner mündet die kombinierte Spülwasser- und Dampfzufuhrleitung 25 in drei unterhalb des Spülaggregats 4 befindlichen Spritzdüsen 27 zur Urinflaschen-Reinigung.

Es stellt sich folgende Wirkungsweise ein:

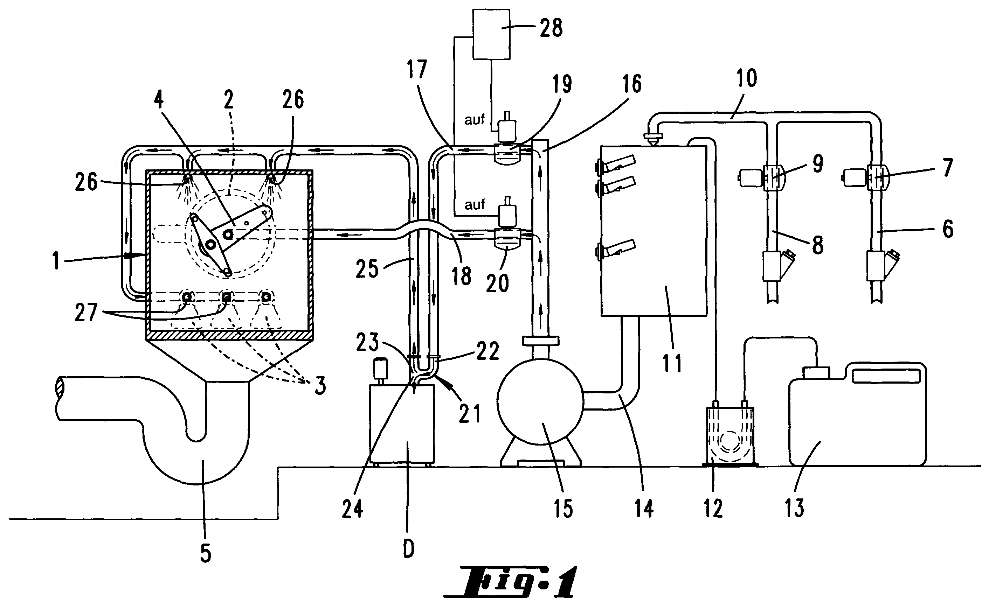

Gemäß der Darstellung in Fig. 1 nimmt die Kammer 1 sowohl eine Bettpfanne 2 als auch drei Urinflaschen 3 auf. Letztere erstrecken sich mit ihrer Öffnung vor den Spritzdüsen 27, während die Bettpfanne 2 dem Spülaggregat 4 zugekehrt ist. Zum Spülen dieses Pflegegeschirrs werden, von einer Zeitsteuereinrichtung 28 veranlaßt, die Magnetventile 19, 20 der Wasserzufuhrleitungen 17, 18 geöffnet, vgl. Fig. 1. Das mit Druck aus dem Wasservorratsbehälter 11 entnommene Spülwasser gelangt hierdurch über die Wasserzufuhrleitung 18 zum Spülaggregat 4, dessen Spritzdüsen die Bettpfanne 2 bestreichen und diese reinigen. Ferner fließt durch die Wasserzufuhrleitung 17 Spülwasser zur Verzweigung 21. Hierbei wird der Dampferzeuger D maximal gefüllt. Es strömt sodann das Spülwasser in Pfeilrichtung durch die kombinierte Spülwasser- und Dampfzufuhrleitung 25 und tritt einerseits an den Dampfdüsen 26 und andererseits an den Spritzdüsen 27 aus, so daß das Pflegegeschirr allseitig gespült wird.

Es kann jedoch auch der Fall auftreten, daß ausschließlich eine Bettpfanne 2 in die Kammer 1 eingesetzt ist. Dann wird das Magnetventil 19 der Wasserzufuhrleitung 17 abgeschaltet, während das der Wasserzufuhrleitung 18 zugeordnete Magnetventil 20 offenbleibt. Das Spülwasser gelangt demzufolge ausschließlich durch die Wasserzufuhrleitung zum Spülaggregat 4, welches bei seinem Umlauf die Bettpfanne 2 reinigt.

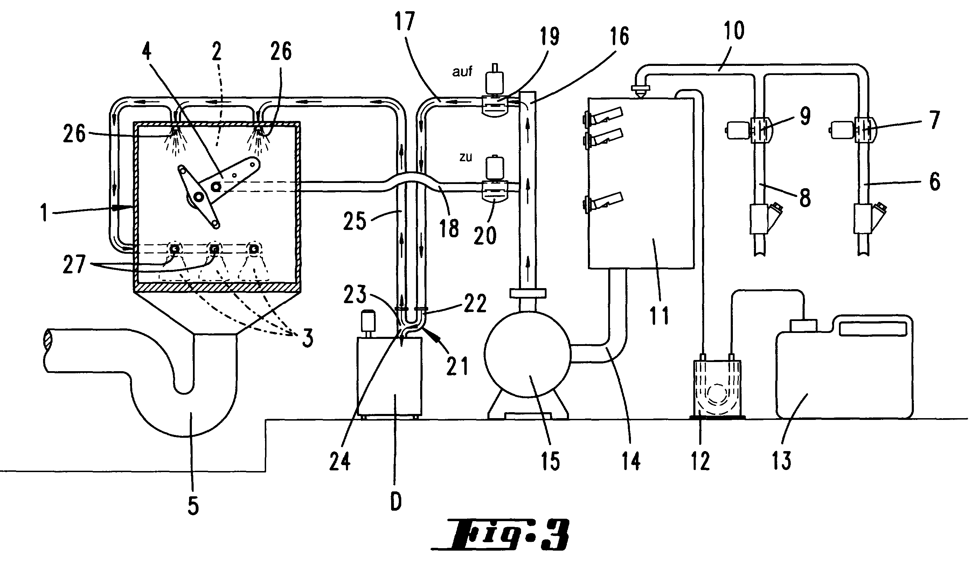

Sodann kann sich der Fall gemäß Fig. 3 einstellen, wobei nur die Reinigung von Urinflaschen 3 angestrebt ist. Dann wird das Magnetventil 20 der Wasserzufuhrleitung 18 geschlossen, während das Magnetventil 19 der Wasserzufuhrleitung 17 geöffnet wird. Über die Verzweigung 21 erfolgt das Befüllen des Dampferzeugers D bis zu seinem maximalen Füllstand und das Durchströmen der Spülwasser- und Dampfzufuhrleitung 25, wodurch die Dampfdüsen 26 sowie die Spritzdüsen 27 spülwasserbespeist werden unter Reinigung der Urinflaschen 3.

Soll die thermische Desinfektion des Pflegegeschirrs - Bettpfanne 2 und Urinflaschen 3 - erfolgen, so sind die Magnetventile 19, 20 der Wasserzufuhrleitungen 17, 18 zu schließen. Der Dampferzeuger D tritt in Aktivität, so daß aus diesem der Dampf durch die kombinierte Spül-wasser- und Dampfzufuhrleitung 25 zu den Dampfdüsen 26 gelangt und dort austritt. Ferner ist es möglich, daß Dampf auch durch die Spritzdüsen 2 austritt, so daß aufgrund des in die Kammer 1 einströmenden Dampfes eventuell am Pflegegeschirr anhaftende Keime mit Sicherheit abgetötet werden.

Alle offenbarten Merkmale sind erfindungswesentlich. In die Offenbarung der Anmeldung wird hiermit auch der Offenbarungsinhalt der zugehörigen/beigefügten Prioritätsunterlagen (Abschrift der Voranmeldung) vollinhaltlich mit einbezogen, auch zu dem Zweck, Merkmale dieser Unterlagen in Ansprüche vorliegender Anmeldung mit aufzunehmen.

## Patentansprüche

1. Spül- und Desinfektionsvorrichtung für Pflegegeschirr, wie z.B. Bettpfannen, Urinflaschen oder dergleichen, mit einer das Pflegegeschirr aufnehmenden Kammer (1), in welche Spülwasser und Dampf zugeführt werden, wobei die Wasserzufuhr magnetventilgesteuert zu- und abschaltbar ist und der Dampf von einem Dampferzeuger (D) erzeugt wird, dadurch gekennzeichnet, daß eine kombinierte Spülwasser- und Dampfzufuhrleitung (25) eine unmittelbar oberhalb des Dampferzeuger (D) angeordnete Verzweigung (21) zum Dampferzeuger (D) besitzt.

2. Spül- und Desinfektionsvorrichtung nach Anspruch 1 oder insbesondere danach, gekennzeichnet durch zwei getrennt voneinander zu- und abschaltbare Wasserzufuhrleitungen (17, 18), von denen die eine (17) die Verzweigung (21) zum Dampferzeuger (D) besitzt und die andere (18) unmittelbar in ein innerhalb der Kammer (1) angeordnetes Spülaggregat (4) mündet.

3. Spül- und Desinfektionsvorrichtung nach einem oder mehreren der vorhergehenden Ansprüche oder insbesondere danach, dadurch gekennzeichnet, daß die eine Wasserzufuhrleitung (17) in Spritzdüsen (21) zur Urinflaschen-Reinigung mündet.

4. Spül- und Desinfektionsvorrichtung nach einem oder mehreren der vorhergehenden Ansprüche oder insbesondere danach, dadurch gekennzeichnet, daß die eine Wasserzufuhrleitung (17) strömongsverbunden ist mit vorzugsweise im oberen Bereich der Kammer (1) befindlichen Dampfdüsen (26).

5. Spül- und Desinfektionsvorrichtung nach einem oder mehreren der vorhergehenden Ansprüche oder insbesondere danach, dadurch gekennzeichnet, daß die Magnetventile (19, 20) von einer elektronischen Zeitsteuereinrichtung (28) betätigt werden.

6. Spül- und Desinfektionsvorrichtung nach einem oder mehreren der vorhergehenden Ansprüche oder insbesondere danach, dadurch gekennzeichnet, daß das Wasser zum Spülen und zur Dampferzeugung einem Wasservorratsbehälter (11) entnommen wird.

7. Spül- und Desinfektionsvorrichtung nach einem oder mehreren der vorhergehenden Ansprüche oder insbesondere danach, dadurch gekennzeichnet, daß die Verzweigung (21) ein U-förmiges Durchflußstück ausbildet, wobei der abflußseitige U-Schenkel (23) U-stegseitig einen in den Dampferzeuger (D) mündenden Stutzen (24) ausbildet.
